Europäisches Patentamt

(19) European Patent Office

Office européen des brevets

(11) Veröffentlichungsnummer : **0 188 819**
**B1**

(12) **EUROPÄISCHE PATENTSCHRIFT**

(45) Veröffentlichungstag der Patentschrift :
23.11.89

(51) Int. Cl.⁴ : **A 61 C 19/04**

(21) Anmeldenummer : 85116631.4

(22) Anmeldetag : 28.12.85

(54) **Vorrichtung zur Messung der Positionen und Bewegungen des Unterkiefers relativ zum Oberkiefer.**

(30) Priorität : 10.01.85 DE 3500605

(43) Veröffentlichungstag der Anmeldung :
30.07.86 Patentblatt 86/31

(45) Bekanntmachung des Hinweises auf die Patenterteilung : 23.11.89 Patentblatt 89/47

(84) Benannte Vertragsstaaten :
AT BE CH DE FR GB IT LI LU NL SE

(56) Entgegenhaltungen :
EP-A- 0 025 201
EP-A- 0 043 149
DE-A- 3 312 245
US-A- 3 259 984

(73) Patentinhaber : Hansen, Markus Dr.
Goethestrasse 46
D-5300 Bonn 1 (DE)

(72) Erfinder : Hansen, Markus Dr.
Goethestrasse 46
D-5300 Bonn 1 (DE)

(74) Vertreter : Selting, Günther, Dipl.-Ing. et al
Deichmannhaus am Hauptbahnhof
D-5000 Köln 1 (DE)

**Beschreibung**

Die Erfindung betrifft eine Vorrichtung zur Messung der Positionen und Bewegungen des Unterkiefers relativ zum Oberkiefer.

Bei einer bekannten Vorrichtung dieser Art (EP-OS 0 025 201) bestehen die Halter aus Kieferplatten, die im Mund des Patienten angeordnet werden. An der Kieferplatte des Oberkiefers ist ein einziger Sender montiert und an der Kieferplatte des Unterkiefers sind mehrere Empfänger verteilt angeordnet. Jeder Empfänger bildet mit dem Sender eine Abstandsmeßeinrichtung. Wenn der Patient den Unterkiefer bewegt, werden die sich verändernden Abstände zwischen jedem Empfänger und dem Sender aufgezeichnet. In einem Artikulator, der Modelle des Unterkiefers und des Oberkiefers enthält, können die Kieferbewegungen unter Steuerung durch die aufgenommenen Daten reproduziert werden. Mit der bekannten Vorrichtung können bei Verwendung von drei Abstandsmeßstrecken nicht alle Kieferbewegungen eindeutig identifiziert werden. Das Meßsystem ist so konzipiert, daß die Kieferbewegungen in einem Artikulator nachvollzogen werden, jedoch ist keine exakte Vermessung der Kiefer- und der Mundbewegungen möglich. Insbesondere die Feststellung und Überprüfung der Gelenkachse zwischen Unterkiefer und Oberkiefer, deren Position sich bei Kaubewegungen verändert, ist am Kopf des Patienten nicht möglich.

Der Erfindung liegt die Aufgabe zugrunde, eine Vorrichtung der eingangs genannten Art zu schaffen, die eine exakte Erfassung und Analyse der Bewegungen des Unterkiefers relativ zum Oberkiefer ermöglicht und dabei sämtliche Freiheitsgrade des Systems eindeutig erfaßt.

Die Lösung dieser Aufgabe erfolgt erfindungsgemäß mit den im Patentanspruch 1 angegebenen Merkmalen.

Bei der erfindungsgemäßen Vorrichtung bilden die Sender und Empfänger ein Multiplexsystem, wobei die Aktivierungssignale der Sender jeweils einen Zeitkanal definieren. Jeder Sender gibt zu Beginn seiner Kanalzeit einen kurzen Impuls aus. Im Laufe der Kanalzeit empfangen die einzelnen Empfänger den Ultraschallimpuls, wobei die Empfangszeit von dem Abstand zwischen dem aktivierten Sender und dem jeweiligen Empfänger abhängt. Innerhalb der Kanalzeit sprechen sämtliche Empfänger auf das von dem aktivierten Sender ausgesandte Signal an. Auf diese Weise erhält man innerhalb einer Kanalzeit eine der Anzahl der Empfänger entsprechende Zahl von Abstandswerten. In der nächstfolgenden Kanalzeit wird ein anderer Empfänger aktiviert und man erhält wiederum eine der Anzahl der Empfänger entsprechende Anzahl von Abstandswerten. Vorzugsweise beträgt die Zahl der Sender, die an dem einen Halter angeordnet sind, und die Zahl der Empfänger, die an dem anderen Halter angeordnet sind, jeweils drei. Bei drei Sendern und Empfängern erhält man neun Abstandsmeßwerte. Da der Unterkiefer sich in Bezug auf den Oberkiefer in sechs Freiheitsgraden bewegen kann (drei translatorische und drei rotatorische Freiheitsgrade), würden sechs Abstandsmessungen ausreichen, um alle Bewegungen in eindeutiger Weise zu erfassen. Bei neun Meßwerten liegt bereits Redundanz vor, die jedoch eine zusätzliche Sicherheit schafft.

In einem Rechner, der die Abstandsmeßwerte verarbeitet, wird die Position des unteren Halters in Bezug auf die als ortsfest angenommene Position des oberen Halters berechnet. Hierbei wird für die Berechnungen in einem Computer ein Koordinatensystem verwendet, das in Bezug auf den oberen Halter ortsfest ist. In diesem Koordinatensystem wird die Position und Ausrichtung des unteren Halters jeweils berechnet. Damit hat der Zahnarzt aber noch nicht die Möglichkeit, die Bewegungen zu ermitteln, die ein bestimmter Punkt des Unterkiefers, z. B. einen Zahn, in Bezug auf einen bestimmten Punkt des Oberkiefers ausführt. Um eine gegenseitige Zuordnung von Zähnen, Knochenteilen und anderen Elementen des Mundes zu ermöglichen, ist gemäß einer bevorzugten Weiterbildung der Erfindung ein frei bewegbarer dritter Halter vorgesehen, der eine Tastspitze aufweist und Sender oder Empfänger trägt, die mit den Empfängern oder Sendern des ersten Halters zusätzliche Abstandsmeßeinrichtungen bilden. Ein Rechner berechnet aus den Werten der zusätzlichen Abstandsmeßeinrichtungen die Positionsdaten der Tastspitze in Bezug auf einen zum ersten Halter ortsfesten Koordinatenpunkt.

Durch den mit der Tastspitze ausgestatteten dritten Halter ist es dem Zahnarzt möglich, dem Rechner bestimmte Punkte des Unterkiefers oder andere Teile des Mundes einzugeben, um nachfolgend z. B. die Bewegungsbahn dieser Punkte bei verschiedenen Mundbewegungen aufzeichnen oder an einem Bildschirm oder auf andere Weise wiedergeben zu lassen. Der dritte Halter, der mit dem ortsfesten ersten Halter zusammenwirkt, ermöglicht es dem Zahnarzt, in den Rechner Positionsdaten von interessierenden Punkten von Unterkiefer und/oder Oberkiefer einzugeben, beispielsweise einen Punkt der Kaufläche eines einzusetzenden künstlichen Zahnes. Werden Oberkiefer und Unterkiefer gegeneinandergedrückt, so kann festgestellt werden, ob die Kaufläche des künstlichen Zahnes die richtige Position einnimmt und die Schließbewegung des Mundes nicht behindert. Erforderlichenfalls kann die Position der Kaufläche korrigiert werden.

Eine andere Anwendungsart des mit der Tastspitze versehenen dritten Halters besteht in der Ermittlung der Gelenkachse, um die der Unterkiefer beim Kauen in Bezug auf den Oberkiefer verschwenkt wird. Diese Gelenkachse ist nicht fest, sondern ihre Lage verändert sich in Abhängigkeit von der Öffnungsstellung des Mundes. Wenn der Patient mit den an seinen Kiefern befestigten Haltern Kaubewegungen ausführt, kann die Lage der Gelenkachse vom Rechner errechnet werden. Der Zahnarzt kann dann den dritten Halter so positionieren, daß die Tastspitze in die Nähe des Backenknochens an eine Stelle zeigt, an der vermutet wird, daß sie in einem bestimmten Öffnungsbereich des Mundes auf der Gelenkachse liegt. Der Rechner überprüft dann, ob die Tastspitze auf der von ihm berechneten Gelenkachse liegt und kann dem Zahnarzt Korrekturwerte angeben. Auf diese Weise kann der Zahnarzt mit Hilfe des Rechners exakt die Lage der Gelenkachse am Kopf des Patienten bestimmen. Dies ist für die spätere Einstellung des Artikulators, in den Gipsmodelle von Oberkiefer und Unterkiefer eingesetzt werden, wichtig, um die Gelenkachse des Artikulators so einzustellen, daß im Artikulator das Gipsmodell des Unterkiefers in derselben Weise bewegt wird wie im Mund des Patienten.

Vorzugsweise sind die Sender oder Empfänger an dem dritten Halter in gleicher Konfiguration und mit gleichen gegenseitigen Abständen angeordnet wie die Sender oder Empfänger des zweiten Halters. Hierdurch wird die Programmierung des Rechners vereinfacht, da die sich auf den dritten Halter beziehenden Abstandssignale in gleicher Weise errechnet werden können wie diejenigen Abstandssignale, die sich auf den zweiten Halter beziehen, welcher am Unterkiefer des Patienten befestigt ist.

Bei der Verwendung von Ultraschallsignalen zur Abstandsmessung ergeben sich Probleme dadurch, daß die Schallgeschwindigkeit von der Temperatur und Feuchtigkeit der Luft beeinflußt wird. Da die Meßstrecken der warmen und feuchten Atmungsluft des Patienten ausgesetzt wird, können Meßfehler auftreten. Zur Eliminierung derartiger Meßfehler ist gemäß einer Weiterbildung der Erfindung vorgesehen, daß an dem ersten Halter und/oder dem zweiten Halter mindestens eine Referenzmeßeinrichtung aus einem Sender und einem Empfänger, die einen konstanten Abstand voneinander haben, angeordnet ist, und daß die Laufzeit der Ultraschallsignale zwischen Sender und Empfänger der Referenzmeßeinrichtung einen Korrekturwert für die Signale der übrigen Abstandsmeßeinrichtungen liefert. Die Referenzmeßeinrichtung besteht aus einem Sender und einem Empfänger, die einen definierten Abstand voneinander haben. Die Laufzeit der Ultraschallsignale zwischen diesem Sender und diesem Empfänger wird als Referenzwert benutzt, um die Signale der übrigen Abstandsmeßeinrichtungen zu korrigieren. Auf diese Weise werden Veränderungen der Schallgeschwindigkeit in der Nähe der Meßapparatur kompensiert.

Im folgenden wird unter Bezugnahme auf die Zeichnungen ein Ausführungsbeispiel der Erfindung näher erläutert.

Es zeigen :

Fig. 1 eine perspektivische Darstellung der Meßapparatur am Patienten,

Fig. 2 eine Darstellung des zweiten Halters und des dritten Halters,

Fig. 3 ein Blockschaltbild der Vorrichtung und

Fig. 4 ein Impulsdiagramm der Sende- und Empfangssignale.

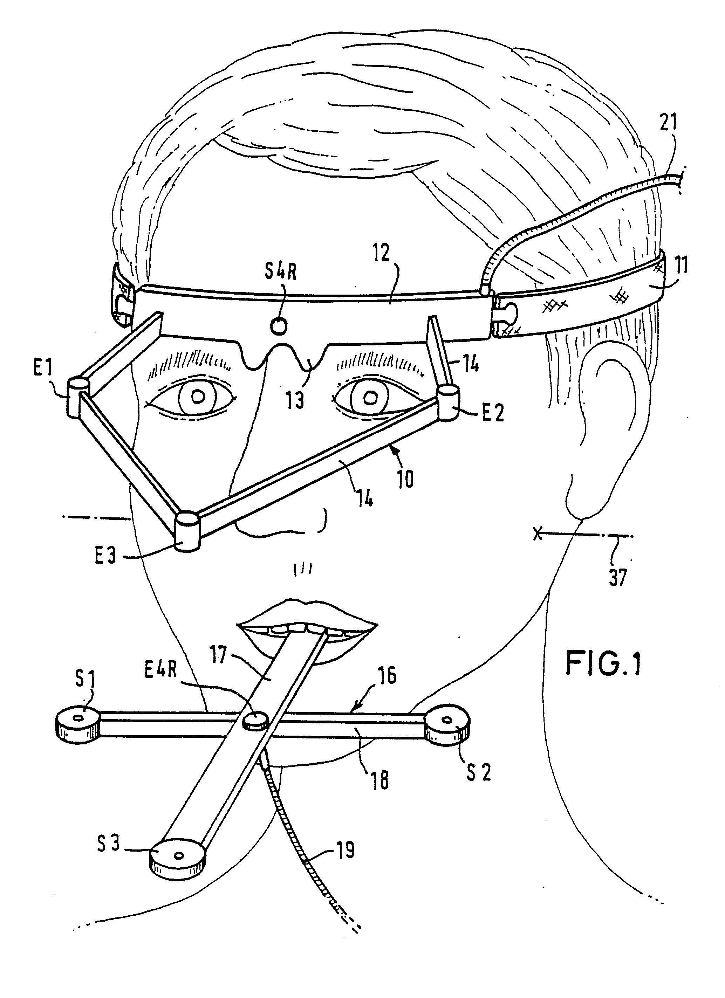

Gemäß Fig. 1 ist am Kopf des Patienten der erste Halter 10 mit einem Stirnband 11, das um den Kopf herumgelegt ist, befestigt. Der Halter 10 besteht aus einem starren Rahmen mit einer gebogenen Leiste 12, die sich flach gegen die Stirn des Patienten legt und mit einem Reiter 13 auf der Nasenwurzel abgestützt ist. Von der Leiste 12 stehen Streben 14 frontal ab. An diesen Streben sind drei Empfänger E1, E2 und E3 befestigt, von denen die beiden seitlichen Empfänger E1 und E2 den gleichen Abstand von der Leiste 12 haben, während der Abstand des mittleren Empfängers E3 von der Leiste 12 größer ist. Die Empfänger E1, E2 und E3 sind — in Draufsicht gesehen — an den Ecken eines horizontalen gleichschenkligen Dreiecks angeordnet. Da der Oberkiefer in bezug auf den Kopf fest ist, nimmt der am Kopf angebrachte erste Halter 10 eine zum Oberkiefer feste Position ein.

Der zweite Halter 16 ist mit einer (nicht dargestellten) Zahnklammer am Unterkiefer des Patienten befestigt. Dieser zweite Halter weist zwei sich rechtwinklig kreuzende Streben 17, 18 auf, von denen die Strebe 17 zwischen den Lippen hindurch aus dem Mund des Patienten herausführt. An den freien Enden der beiden Streben 17 und 18 sind die drei Sender S1, S2 und S3 befestigt. Die Sender sind vorzugsweise an den Eckpunkten eines Dreiecks angeordnet, das mit dem Dreieck, das die Empfänger E1, E2 und E3 bilden, kongruent ist.

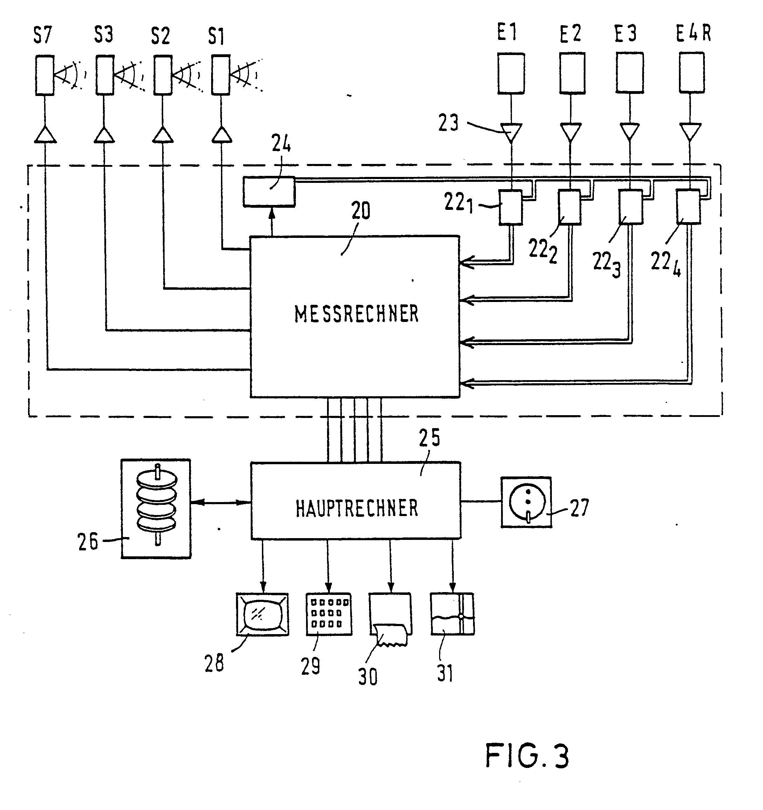

Die Sender S1, S2 und S3 sind über Kabel 19 mit dem Meßrechner 20 (Fig. 3) verbunden und die Empfänger E1, E2 und E3 sind ebenfalls über Kabel 21 mit dem Meßrechner verbunden.

Die Sender S1, S2 und S3 werden nacheinander aktiviert, um einen kurzen Ultraschallimpuls 21 zu erzeugen, der aus einer Druckwelle, deren Anstiegsflanke kürzer als 10 $\mu$s sein sollte, besteht. Wenn der Sender 1 den Ultraschallimpuls 21 aussendet, wird dieser Impuls von den Empfängern E1, E2 und E3 in unterschiedlichen zeitlichen Abständen empfangen, die von der Entfernung des jeweiligen Empfängers von dem Sender S1 abhängen.

Sobald einer der Sender aktiviert worden ist, liefert der Meßrechner 20 an einen Zähler 24 einen Impulstakt von 8 MHz, um den Zählerstand dieses Zählers von Null aus hochzuzählen. Der Zählerstand des Zählers 24 wird an die Eingänge von Halteschaltungen $22_1$, $22_2$, $22_3$ übertragen, die den einzelnen Empfängern E1, E2 und E3 zugeordnet sind. Wenn einer der Empfänger E1 den Ultraschallimpuls empfängt, liefert er über einen Verstärker 23 einen Impuls an die zugehörige Halteschaltung, die daraufhin den Zählwert des Zählers 24 festhält. Auf diese Weise enthält am Ende eines Meßintervalls jede Halteschaltung einen Zählerstand, der der Laufzeit der Ultraschallsignale zwischen dem Sender und dem betreffenden Empfänger proportional ist. Die Inhalte der Halteschaltungen werden anschließend in den Meßrechner 20 eingegeben.

Der Meßrechner 20 ist an den Hauptrechner 25 angeschlossen, der die Abstandsdaten empfängt und durch arithmetische Umrechnung die Position des Unterkiefers in Bezug auf ein Koordinatensystem, das ortsfest zum Oberkiefer ist, berechnet. An den Hauptrechner 25 sind mehrere Peripheriegeräte angeschlossen, z. B. ein Plattenspeicher 26, eine Disketteneinheit 27, ein Bildschirmgerät 28, eine Eingabetastatur 29, einen Drucker 30 und ein Plotter 31.

Bei dem beschriebenen Ausführungsbeispiel steht ein Intervall von 2 ms zur Verfügung, in dem alle Empfänger den von einem Sender ausgesandten Ultraschallimpuls empfangen. Da die Laufzeit der Ultraschallimpulse kleiner ist als 20 ms, steht am Ende eines jeden Intervalls genügend Zeit zur Verfügung, um die Abstandsdaten aus den Halteschaltungen 22 in den Meßrechner 20 zu übernehmen und um verschiedene Rechenoperationen durchzuführen.

An dem zweiten Halter 16, an dem sich die Sender S1, S2 und S3 befinden, ist zusätzlich ein Empfänger E4R angebracht, der im Kreuzungspunkt der Streben 17 und 18 angeordnet ist. Dieser Empfänger E4R bildet mit einem der Sender desselben Halters 16 eine Referenzmeßstrecke. Er empfängt ebenfalls die Signale aller drei Sender S1, S2 und S3, wobei jedoch nur das Signal eines dieser Sender ausgewertet werden muß. Die Halteschaltung $22_4$ dieses Empfängers E4R ist in gleicher Weise gesteuert wie die Halteschaltungen der übrigen Empfänger, und sie liefert an den Meßrechner 20 einen Wert, der der festen Entfernung des Empfängers E4R von einem der Sender S1, S2 oder S3 entspricht. Aus diesem Wert wird durch Vergleich oder durch Verhältnisbildung mit einem Wert, der der normalen Laufzeit zwischen diesem Sender und diesem Empfänger entspricht, ein Korrekturwert ermittelt, um die übrigen Abstandswerte zu korrigieren. Auf diese Weise wird die Schallgeschwindigkeit in der Nähe des Mundes des Patienten in Abhängigkeit von den jeweils herrschenden augenblicklichen Bedingungen ermittelt und bei der Messung berücksichtigt.

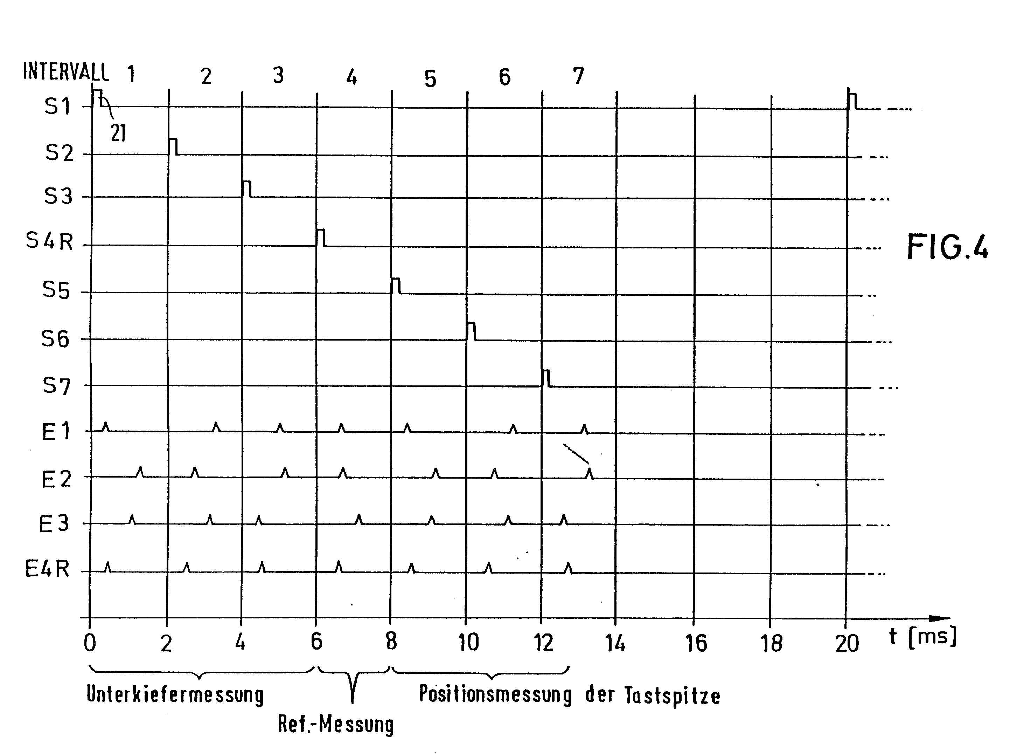

Alternativ oder zusätzlich ist an dem ersten Halter 10, der die Empfänger E1, E2 und E3 trägt, ein Sender S4R befestigt, dem in dem Impulsdiagramm der Fig. 4 ein eigenes Intervall von 2 ms zugeordnet ist. Die Laufzeit des Ultraschallsignals von dem Sender S4R zu einem der Empfänger E1, E2 oder E3 wird ermittelt, um hieraus einen Korrekturwert für die übrigen Laufzeitsignale zu gewinnen.

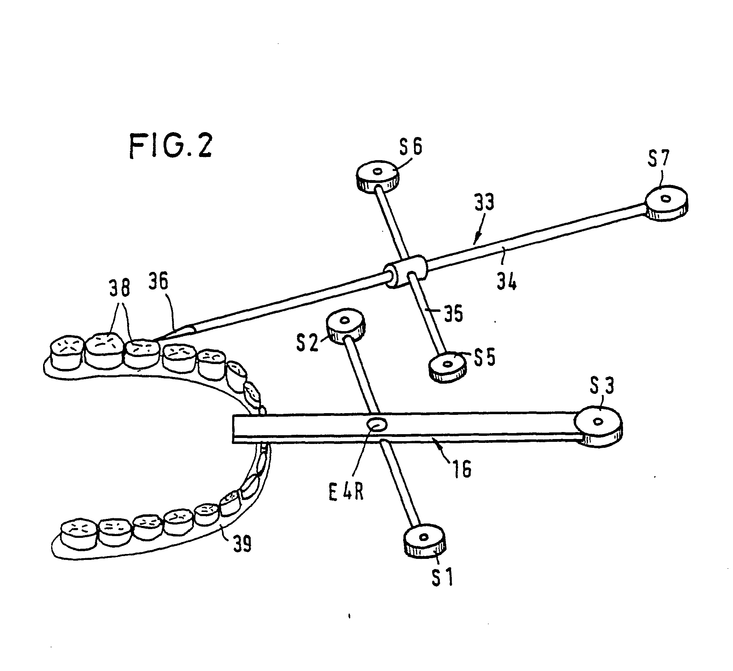

Mit der bisher beschriebenen Einrichtung können Positionsänderungen des Unterkiefers in Bezug auf den Oberkiefer erfaßt werden, jedoch können beispielsweise die Positionen der Zähne des Unterkiefers nicht in Bezug auf die Positionen der Zähne des Oberkiefers bestimmt werden. Gemäß Fig. 2 ist ein dritter Halter 33 vorgesehen, der aus zwei sich kreuzenden Streben 34, 35 besteht. An dem beiden Enden der Strebe 35 befinden sich zwei Sender S5 und S6 und an dem einen Ende der Strebe 34 befindet sich ein dritter Sender S7. Das andere Ende der Strebe 34 ist als Tastspitze 36 ausgebildet. Der dritte Halter 33 ist nicht fest am Körper des Patienten angebracht, sondern dieser Halter kann von dem Zahnarzt frei geführt werden, um die Tastspitze 36 an beliebige Punkte anzusetzen, beispielsweise an einen Zahn. Jedem der Sender S5, S6 und S7 ist ebenfalls ein Intervall von 2 ms zugeteilt. In diesem Intervall werden die Abstände des betreffenden Senders zu jedem der Empfänger E1, E2 und E3 des festen Halters 10 ermittelt. Aus diesen neuen Abstandswerten bestimmt der Rechner die Position der Tastspitze 36 in Bezug auf das Koordinatensystem, das relativ zu dem ersten Halter 10 fest ist. Der Zahnarzt kann also beispielsweise mit der Tastspitze 36 auf einen Punkt im Munde des Patienten zeigen und die Positionsdaten dieses Punktes in den Rechner eingeben. Danach zeigt der Zahnarzt mit der Tastspitze 36 auf einen anderen Punkt im Munde des Patienten und gibt die Positionsdaten diesen zweiten Punktes ebenfalls in den Rechner ein. Wenn anschließend die Bewegungen des Unterkiefers registriert werden, kann in Form einer Kurve dargestellt werden, wie sich beide Meßpunkte relativ zueinander bewegen.

Eine weitere Möglichkeit der Verwendung des zweiten Halters 33 besteht darin, die Kauachse 37 zu kontrollieren, um die herum der Unterkiefer beim Kauen relativ zu dem Oberkiefer geschwenkt wird. Die Position dieser Kauachse 37 (Fig. 1) ist nicht fest, sondern die Kauachse verschiebt sich in Abhängigkeit von der Öffnungsstellung des Mundes. Der Zahnarzt kann mit der Tastspitze 36 auf einen Punkt in der Nähe des Backenknochens zeigen, von dem er meint, daß dieser Punkt auf der Kauachse 37 liegt. In dem Computer ist die Lage der Kauachse 37 ermittelt worden. Der Computer kann nun prüfen, ob die Tastspitze sich auf der Kauachse befindet oder welchen Abstand sie von dieser hat. Auf diese Weise kann der Zahnarzt durch Bewegen der Tastspitze außerhalb des Mundes des Patienten die Lage der Kauachse 37 bei einer bestimmten Öffnungsstellung des Mundes ermitteln und dem Zahntechniker entsprechende Anweisungen für die Einstellung des Artikulators geben. Anstelle des Halters 33 kann für die Bestimmung der Kauachse auch ein modifizierter Halter benutzt werden, bei dem das eine Ende der Strebe 34 abgewinkelt ist.

Das in Fig. 4 dargestellte Impulsdiagramm zeigt, daß die ersten drei Meßintervalle von jeweils 2 ms den Sendern S1, S2 und S3 zugeteilt sind, um die Position des zweiten Halters 16 in Bezug auf den ersten Halter 10 zu ermitteln. In dem vierten Intervall erfolgt die Referenzmessung, also die Ermittlung des Abstandswertes zwischen dem Sender S4R und einem der Empfänger desselben Halters 10, beispielsweise dem Empfänger E3. In dem fünften bis siebten Intervall erfolgt die Positionsmessung des dritten Halters 33 bzw. der Tastspitze 36, in dem die Sender S5, S6 und S7 nacheinander aktiviert werden. Ein derartiger Meßzyklus erfordert 14 ms. An den Meßzyklus kann sich eine Pause von 6 ms anschließen und danach wird der Meßzyklus wiederholt. Auf diese Weise kann das System selbst schnellen Bewegungen des Unterkiefers folgen, weil jeder Meßwert fünfzigmal in der Sekunde aufgenommen wird.

In Fig. 2 ist eine Zahnklammer 39 dargestellt, an der der zweite Halter 16 befestigt ist. Diese Zahnklammer 39 wird am Unterkiefer des Patienten befestigt. Die Zähne des Unterkiefers sind mit 38 bezeichnet.

**Patentansprüche**

1. Vorrichtung zur Messung der Positionen und Bewegungen des Unterkiefers relativ zum Oberkiefer, mit einem am Kopf des Patienten in fester Zuordnung zum Oberkiefer zu befestigenden starren ersten Halter (10), einem am Unterkiefer zu befestigenden starren zweiten Halter (16) und mit mehreren Abstandsmeßeinrichtungen mit jeweils einem Sender und einem Empfänger, die an den Haltern (10, 16) angebracht sind und zusammen mit einem Rechner (20, 25) die Positionen der Halter relativ zueinander ermitteln, wobei an dem einen Halter (10) mindestens drei Empfänger (E1, E2, E3) befestigt sind, dadurch gekennzeichnet, daß an dem anderen Halter (16) mindestens drei verteilt angeordnete nacheinander aktivierbare Sender (S1, S2, S3) für Ultraschallsignale befestigt sind, daß jeder Empfänger (E1, E2, E3) auf die Ultraschallsignale aller Sender (S1, S2, S3) des anderen Halters (16) anspricht, und daß die Intervalle, in denen die Sender (S1, S2, S3) nacheinander aktiviert werden, größer sind als die maximale Laufzeit der Ultraschallsignale zwischen einem Sender und den Empfängern.

2. Vorrichtung nach Anspruch 1, dadurch gekennzeichnet, daß ein frei bewegbarer dritter Halter (33) vorgesehen ist, der eine Tastspitze (36) aufweist und Sender (S5, S6, S7) oder Empfänger trägt, die mit den Empfängern (E1, E2, E3) oder Sendern des ersten Halters (10) zusätzliche Abstandsmeßeinrichtungen bilden, und daß ein Rechner (20, 25) aus den Werten der zusätzlichen Abstandsmeßeinrichtungen die Positionsdaten der Tastspitze (36) in Bezug auf einen zum ersten Halter (10) ortsfesten Koordinatenpunkt berechnet.

3. Vorrichtung nach Anspruch 2, dadurch gekennzeichnet, daß die Sender (S5, S6, S7) an dem dritten Halter (33) in gleicher Konfiguration und mit gleichen gegenseitigen Abständen angeordnet sind wie die Sender (S1, S2, S3) oder Empfänger des zweiten Halters (16).

4. Vorrichtung nach einem der Ansprüche 1 bis 3, dadurch gekennzeichnet, daß an dem ersten Halter (10) und/oder dem zweiten Halter (16) mindestens eine Referenzmeßeinrichtung aus einem Sender (S4R ; S1, S2, S3) und einem Empfänger (E1, E2, E3 ; E4R), die einen konstanten Abstand voneinander haben, angeordnet ist und daß die Laufzeit der Ultraschallsignale zwischen Sender und Empfänger der Referenzeinrichtung einen Korrekturwert für die Signale der übrigen Abstandsmeßeinrichtungen liefert.

**Claims**

1. An apparatus for measuring the positions and movements of the lower jaw relative to the upper jaw, comprising a rigid first holder (10) to be secured to the patient's head in a fixed relationship to the upper jaw, a rigid second holder (16) to be secured to the lower jaw and a plurality of distance measuring devices including a transmitter and receiver, respectively, which are provided at the holders (10, 16) and determine together with a calculator (20, 25) the positions of the holders relative to each other, at least three receivers (E1, E2, E3) being mounted on one holder (10), characterized in that at least three successively activatable transmitters (S1, S2, S3) for ultrasonic signals are distributed over and fixed at the other holder (16), that each receiver (E1, E2, E3) is responsive to the ultrasonic signals from all transmitters (S1, S2, S3) of the other holder (16) and that the intervals at which the transmitters (S1, S2, S3) are successively activated, are longer than the maximum transit time of the ultrasonic signals between a transmitter and the receivers.

2. The apparatus according to claim 1, characterized by a freely movable third holder (33) comprising a scanning tip (36) and carrying transmitters (S5, S6, S7) or receivers forming additional distance measuring devices with the receivers (E1, E2, E3) or the transmitters of the first holder (10), and a calculator (20, 25) calculating from the values of the additional distance measuring devices position data of the scanning tip (36) relative to a point of coordinates being stationary relative to the first holder (10).

3. The apparatus according to claim 2, characterized in that the configuration and mutual relationship of the transmitters (S5, S6, S7) of the third holder (33) are similar to those of the transmitters (S1, S2, S3) or receivers of the second holder (16).

4. The apparatus according to one of claims 1 to 3, characterized in that at least one reference measuring unit is provided at the first holder (10) and/or the second holder (16), including a transmitter (S4R ; S1, S2, S3) and a receiver (E1, E2, E3 ; E4R), mutually spaced at a constant distance, and that the transit time of the ultrasonic signals between transmitter and receiver of the reference unit provides a correction value for the signals of the remaining distance measuring means.

**Revendications**

1. Dispositif pour mesurer les positions et les mouvements de la mâchoire inférieure par rapport à la mâchoire supérieure, comportant un premier support rigide (10) destiné à être attaché à la tête du patient, selon une disposition fixe par rapport à la mâchoire supérieure, un second support rigide (16) destiné à être attaché à la mâchoire inférieure et plusieurs mécanismes de mesure de distance, comprenant à chaque fois un émetteur et un récepteur, qui sont rapportés sur les supports (10, 16) et déterminent ensemble, avec un calculateur (20, 25), les positions des supports l'un par rapport à l'autre, au moins trois récepteurs (E1, E2, E3) étant fixés sur le premier support (10), caractérisé, en ce que sur le second support (16) sont fixés au moins trois émetteurs (S1, S2, S3) de signaux ultrasoniques qui sont disposés dispersés et peuvent être activés l'un après l'autre, en ce que chaque récepteur (E1, E2, E3) réagit aux signaux ultrasoniques de tous les émetteurs (S1, S2, S3) de l'autre support (16), et en ce que les intervalles de temps dans lesquels les émetteurs (S1, S2, S3) sont activés l'un après l'autre sont plus grands que le temps maximal de parcours des signaux ultrasoniques entre un émetteur et les récepteurs.

2. Dispositif selon la revendication 1, caractérisé en ce qu'il est prévu un troisième support (33) qui peut se déplacer librement et qui présente une pointe à palper (36), et porte des émetteurs (S5, S6, S7) ou des récepteurs qui forment, avec les récepteurs (E1, E2, E3) ou avec les émetteurs du premier support (10), des mécanismes supplémentaires de mesure de distance, et en ce qu'à partir des valeurs des mécanismes supplémentaires de mesure des distances, un calculateur (20, 25) calcule les données de position de la pointe à palper (36) par rapport à un point de coordonnées fixes par rapport au premier support (10).

3. Dispositif selon la revendication 2, caractérisé en ce que les émetteurs (S5, S6, S7) sont disposés sur le troisième support (33) dans la même configuration et avec les mêmes distances mutuelles que les émetteurs (S1, S2, S3) ou les récepteurs du second support (16).

4. Dispositif selon l'une des revendications 1 à 3, caractérisé en ce que sur le premier support (10) et/ou sur le second support (16) est disposé au moins un mécanisme de mesure de référence constitué d'un émetteur (S4R ; S1, S2, S3) et d'un récepteur (E1, E2, E3 ; E4R), qui sont à une distance constante l'un de l'autre ; et en ce que le temps de parcours des signaux ultrasoniques entre les émetteurs et les récepteurs du mécanisme de référence fournit une valeur correctrice pour les signaux des autres mécanismes de mesure de distance.

FIG.1

FIG.2

FIG.3

FIG.4

INTERVALL 1   2   3   4   5   6   7

Unterkiefermessung    Ref.-Messung    Positionsmessung der Tastspitze

EP 0 188 819 B1